Europäisches Patentamt

European Patent Office

Office européen des brevets

(19)

(11) Veröffentlichungsnummer: **0 085 747**
**B2**

(12) **NEUE EUROPÄISCHE PATENTSCHRIFT**

(45) Veröffentlichungstag der neuen Patentschrift:
**30.05.90**

(21) Anmeldenummer: **82109362.2**

(22) Anmeldetag: **09.10.82**

(51) Int. Cl.⁵: **A 61 K 39/395**

(54) **Intravenös verabreichbares humanes Immunglobulin und Verfahren zu dessen Herstellung.**

(30) Priorität: **08.02.82 CH 741/82**

(43) Veröffentlichungstag der Anmeldung:
**17.08.83 Patentblatt 83/33**

(45) Bekanntmachung des Hinweises auf die
Patenterteilung:
**14.05.86 Patenblatt 86/20**

(45) Bekanntmachung des Hinweises auf die
Entscheidung u̅ber den Einspruch:
**30.05.90 Patentblatt 90/22**

(84) Benannte Vertragsstaaten:
**AT BE CH DE FR GB IT LI NL SE**

(56) Entgegenhaltungen:
EP-A-0 025 719
EP-A-0 092 186
DE-A-2 500 076
DE-A-2 827 027
GB-A-1 372 953
US-A-4 126 605

U.E. Nydegger "Immunohemotherapy - A Guide
to Immunoglobulin Prophylaxis and Therapy",
1981, S.113-130 u. S. 153-154f.

(73) Patentinhaber: **Schweizerisches Serum- und
Impfinstitut und Institut zur Erforschung der
Infektionskrankheiten
Rehhagstrasse 79
CH-3018 Bern (CH)**

(72) Erfinder: **Rodolphe, Fritsché, Dr.
Chemin du champ 6
CH-1723 Marly (CH)**
Erfinder: **Norbert, Chariatte, Dr.
Thunstrasse 163
CH-3074 Muri bei Bern (CH)**

(74) Vertreter: **Zutter, Hans Johann Niklaus
EPROVA AG Forschungsinstitut Im
Laternenacker 5
CH-8200 Schaffhausen (CH)**

(56) Entgegenhaltungen:
**Alving et al, "Immunoglobulins, Characteristics
and Uses of Intravenous Preparations", US.
Department of Health and Human Services,
Public Health Service Washington 1980, R.M.
Condie, "Preparation and Intravenous Use of
Undenatured Human IgG", S. 179-193**

**Develop. biol. Standard., Bd. 44, 1979, Seiler et
al, "Comparison of several in vitro Assay
Methods for the Quantitative Determination of
Complement Consumption ('Binding') by
Gammaglobulin Preparations", S. 153-163**

EP 0 085 747 B2

# EP  0 085 747  B2

**(56) Entgegenhaltungen:**

A. Morell und E. Nydegger, "Clinical use of intravenous immunoglobulins", Proceedings of a conference held at Interlaken 15.-18.

September 1985, Academic Press 1986
A.D. Friesen et al, "Column Ion Exchange Chromatographic Production of Human Immune Serum Globulin for Intravenous Use", Vox Sanguinis 48, 1985, S. 201-212

P. Gronski et al, "Quality Criteria for i.v.-Immunoglobulins: Importance of Tests and Product Properties", Behring Inst. Mitt., Nr. 80, 16-30, 1986

Wissenschaftliche Tabellen Geigy, 8. Auflage, Basel 1979, Band 2: Physikalische Chemie/Blut/Humangenetik/Stoffwechsel von Xenobiotika, S. 124, 125 u. 139

Informal Meeting on Intravenous Immunoglobulins (Human), Genf, 29.11.-01.12.1982, WHO/BS/83.1396

Schweiz. Serum- & Impfinstitut Bern, Proteinchemie/Forschungsgruppe, "Herstellung von intravenös applizierbarem Immunglobulin G", Protokoll IgG L250687

Schweiz. Serum- & Impfinstitut Bern, Proteinchemie/Forschungsgruppe, "Herstellung eines Immunglobulin-Präparates", Protokoll IgG L090787, 09.07.1987

Schweiz. Serum- & Impfinstitut Bern, Proteinchemie, Versuchsprotokoll Präparat H (i.v. Immunglobulin)

**Beschreibung**

Die vorliegende Erfindung betrifft ein neues intravenös verabreichbares natives, chemisch unverändertes und enzymatisch nicht abgebautes, stabiles Immunglobulin, das in vitro keine antikomplementäre Aktivität aufweist sowie das Verfahren zu dessen Herstellung.

Humane Immunglobuline spielen eine bedeutende Rolle bei der Prophylaxe und der Behandlung von infektiösen Krankheiten und Antikörpermangelzuständen Gammaglobuline werden zum Beispiel zur Prophylaxe von Infektionen angewendet, welche viralen Ursprungs sind wie Hepatitis, Masern, Röteln, Mumps, Tollwut oder auf Bakterien zurückzuführen sind wie Tetanus, Diphtherie, Keuchhusten Bei antibiotika-resistenten Infektionen verursacht z.B. durch Staphylokokken, Escherichia Coli, Pseudomonas u.a.m. werden Gammaglobuline therapeutisch eingesetzt. Spezifische Immunglobuline (IgG) werden auch zur Prophylaxe von Rhesusinkompatibilitäten benutzt.

Ausserdem ist die Behandlung mittels Gammaglobulinen zum Schutze vor Infektionen bei immundefizienten Patienten, die an Agamma- oder Hypogammaglobulinämien leiden, sehr wichtig.

Die übliche intramuskuläre Verabreichung hat jedoch einige schwerwiegende Mängel:

1. Es kann nur eine beschränkte Menge von höchstens 10 ml injiziert werden.

2. Die Resorption durch den Organismus ist stark verzögert. R. Martin du Pan et al., Blut 5, 104 (1959) bemerkten beispielsweise, dass nach 5 Tagen noch 35 bis 40% des Immunglobulins an der Injektionsstelle zu finden war.

3. Ein grosser Teil des Immunglobulins wird durch Proteolyse an der Injektionsstelle abgebaut. Vgl. dazu S. Barandun et al., Vox Sanguinis, 28, 1571—75 (1975).

Im Gegensatz dazu führt die intravenöse Injektion oder Infusion rasch zu einem hohen Immunglobulin-Blutspiegel, wie er z.B. für die Behandlung von septisch-toxischen Infektionen notwendig ist.

Für die intravenöse Applikation darf jedoch nicht ein Immunglobulin verwendet werden, das durch bekannte Fraktionierungsverfahren aus Blutplasma, Serum oder Placenten hergestellt worden ist und sich nur zur intramuskulären Applikation eignet. Vergleiche dazu Cohn et al., J. Amer. chem. Soc. 68, 459 (1946), A.J.L. Strauss et al., J. Immunol. 93, 24 (1964), A. Horejsi et al,. Acta Med. Scand. 155, 65 (1956), A. Polson et al., Biochem. Biophys. Acta 82, 463 (1964).

Bei intravenöser Verabreichung solcher Immunoglobuline treten bei etwa 15 bis 30% der Patienten anaphylaktoide Unverträglichkeitsreaktionen auf (C.A. Janeway et al., New Engl. J. Med. 278, 919 (1968). Bei immundefizienten Patienten die eine IgG-Zufuhr besonders dringend benötigen, beträgt die Rate dieser anaphylaktoiden Unverträglichkeiten etwa 90%. Es gibt bis heute noch keine restlos befriedigende Erklärung für diese Reaktionen. Man weiss aber, dass sie mit allergrösster Wahrscheinlichkeit durch komplementbindende IgG-Aggregate ausgelöst werden, die in handelsüblichen Immunglobulin-Präparaten häufig nachweisbar sind. Für diese Annahme spricht, dass ein direkter Zusammenhang zwischen den klinischen Unverträglichkeitsreaktionen, dem Anteil an zirkulierendem Komplement und der in vitro bestimmten antikomplementären Aktivität der Immunoglobuline festgestellt wurde. Haupziel bei der Zubereitung intravenös verabreichbarer Immunoglobuline ist deshalb die Entfernung derer antikomplementären Aktivität (S. Barandun et al., Vox Sanguinis 7, 157—174 (1962)).

Zur Verminderung der antikomplementären Aktivität der Immunglobuline (IgG) sind bis jetzt mehrere Möglichkeiten bekannt:

Es ist vorgeschlagen worden, die IgG-Aggregate durch Ultrazentrifugation oder chromatographische Auftrennung zu entfernen. Die nach diesen Methoden erhaltenen Präparate sind jedoch unstabil und werden rasch wieder antikomplementär, wahrscheinlich infolge der Reaggregation der Monomeren (S. Barandun et al., Vox Sanguinis 7, 157, (1962)).

Nach der österreichischen Patentschrift 359 640 kann die antikomplementäre Aktivität durch Mischen mit Albumin oder Serum reduziert werden. Die nach diesen Verfahren erhaltenen Präparate können jedoch nicht mehr als Immunglobuline bezeichnet werden, da zur genügenden Herabsetzung der antikomplementäten Aktivität die Zugabe von Albumin oder Serum so hoch wird, dass der IgG-Anteil in der Gesamtproteinmenge zu gering wird.

Die Beseitigung der IgG-Aggregate wurde auch durch Adsorption mittels Aktivkohle (M. Steinbruch, Vox Sanguinis 13, 103 (1967)), mit Stärke, mit Silikaten (Deustche Offenlegungsschrift 26 58 334), sowie durch Fällung mit Polyethylenglykolen (Deutsche Offenlegungsschrift 27 51 717) versucht. Keine dieser Methoden erlaubt eine vollständige Beseitigung der antikomplementären Aktivität.

Durch partiellen enzymatischen Abbau von Immunglobulin wurden Präparate ohne komplementbindende Aktivität erhalten, da proteolytische Enzyme wie Pepsin und Plasmin vorzugsweise den Fc-Teil des IgG-Moleküls, welcher die komplementbindende Domäne dieses Moleküls enthält, abspalten oderzerstören.

Der grosse Nachteil dieser Methode ist jedoch die vollständige Aufhebung der biologischen Funktionen, die mit dem Fc-Teil des IgG-Moleküls verbunden sind, wie zum Beispiel die zytophile Aktivität, die Opsonisation oder die Zytolyse (Bakteriolyse), die alle einen unversehrten Fc-Teil benötigen, der sich an Fc-Rezeptoren binden kann. Ausserdem entstehen bei der enzymatischen Spaltung des Immunglobulin-Moleküls Fab und $F(ab')_2$-Fragmente, die das spezifische Antigen zwar zu binden vermögen, aber nur eine sehr kurze biologische Halbwertszeit, nämlich 18 bis 24 Stunden anstatt 18 bis 22 Tage wie das vollständige IgG-Molekül, aufweisen.

Trotz dieser schwerwiegenden Nachteile sind mehrere, mit Enzymen gespaltene, i.v. applizierbare Immunglobulin-Präparate im Handel:

In der französichen Patentschrift. 2.382M sind mit Pepsin behandelte Produkte beschrieben, welche zu ca. 80% aus F(ab')$_2$-Fragmenten bestehen. Nur 3 bis 5% IgG-Moleküle haben der Proteolyse widerstanden. Diese Präparate haben keine antikomplementäre Aktivität und die F(ab')$_2$-Fragmente vermögen Toxine und Viren zu neutralisieren.

Mit Plasmin behandelte IgG-Präparate sind z.B. in der deutschen Offenlegungsschrift 27 52 694 beschrieben. Sie enthalten etwa 30 bis 40% intaktes IgG (Subklassen 2 und 4), das nicht proteolytisch gespalten wird. Die komplementbindende Aktivität dieser präparate ist schwach (20 bis 50 mg/ml/2CH$_{50}$).

Durch Säurebehandlung bei pH 4 während 24 Stunden bei 37 Grad Celsius kann z.B. nach S. Barandun et al., Vox Sanguinis 7, 157 (1962) die antikomplementäre Aktivität der IgG stark herabgesetzt werden. Die erhaltenen Präparate bestehen aus 85 bis 90% monomerem IgG und 10 bis 15% IgG-Aggregaten. Die komplementbindende Aktivität dieser Produkte ist schwach (50 bis 70 mg/ml/2CH$_{50}$). Ihre biologische Halbwertszeit ist jedoch auf etwa 14 Tage verkürzt und die Präparate sind während der Lagerung nicht stabil und ihre antikomplementäre Aktivität nimmt wieder zu. Ein stabiles, lyophilisiertes Präparat, das nach dieser Methode hergestellt worden ist, ist seit kurzem im Handel erhältlich.

Es wurde auch vielfach versucht und vorgeschlagen, intravenös applizierbare IgG-Präparate durch Einwirkung von reaktiven Chemikalien zu erhalten.

a) Mit β-Propiolacton werden die Komplement-Rezeptoren auf dem Fc-Teil des IgG blokkiert. Die so erhaltenen Produkte binden kein Komplement mehr und bestehen zu 90% aus Monomeren, ihre biologische Halbwertszeit ist jedoch auf 4 bis 12 Tage herabgesetzt. Vergleiche dazu: europäische Patentanmeldung 13 901 S. Barandun et al., Monograph. Allergy, 9, 39—60 (Karger, Basel 1975).

b) Durch Reduktion und Sulfonierung der Disulfid-Brücken des IgG-Moleküls kann nach T. Yamanaka et al., Vox Sanguinis 37, 14—20 (1979) die antikomplementäre Aktivitätebenfalls stark vermindert werden.

c) Durch Reduktion und Alkylierung gemäss. einem Vorschlag von D.D. Schröder et al., Vox Sanguinis 40, 383—394 (1981) oder durch Amidierung gemäss deutscher Offenlegungsschrift 24 42 655 kann man zu intravenös verabreichbaren IgG-Präparaten kommen.

Veränderungen der Molekularstruktur und das Auftreten neuer antigener Determinanten auf dem IgG-Molekül können durch diese chemischen Eingriffe nicht ausgeschlossen werden.

Bisher gibt es kein ideales Immunglobulin-Präparat für die intravenöse Verabreichung. Entweder werden die IgG-Moleküle durch enzymatischen Abbau unter Verlust der vom Fc-Fragment abhängigen Eigenschaften oder durch chemischen Eingriff unter Veränderung der Molekülstruktur modifiziert, wobei gewöhnlich auch noch die biologische Halbwertszeit verkürzt wird, oder es bleibt, wenn mit vorwiegend physikalischen Massnahmen gearbeitet wurde, noch eine gewisse antikomplementäre Aktivität im IgG-präparat übrig.

Ziel der vorliegenden Erfindung ist, Immunglobulin derart zu reinigen und zu behandeln, dass ein stabiles Produkt erhalten wird, dessen native Molekularstruktur unangetastet bleibt, das damit die ursprüngliche Antikörperaktivität bewahrt und das keine in vitro nachweisbare antikomplementäre Aktivität enthält und demnach ohne Gefahr auch bei den besonders gefärdeten immundefizienten Patienten intravenös applizierbar ist.

Dieses Ziel ist gemäss vorliegender Erfindung durch ein Verfahren erreicht worden, welches in einer Kombination von drei aufeinanderfolgenden und aufeinander abgestimmten Etappen besteht, wobei in jeder Etappe die antikomplementäre Aktivität der Immunglobuline vermindert wird.

Die erste Etappe besteht in einer grundlegenden Trennung von monomerem Immunglobulin G und dessen Aggregaten mit Hilfe eines Ionenaustauschers, indem man die IgG-Monomeren selektiv eluiert, während die IgG-Aggregate auf dem Ionenaustauscher gebunden bleiben. Die eluierten Monomere binden das Komplement nur noch schwach, sind aber sehr unstabil und werden rasch wieder antikomplementär, wenn sie nicht stabilisiert werden.

Diese Stabilisierung wird in einer zweiten Etappe nach an sich bekannter Methode von S.I. Miekka et al., Vox Sanguinis 29, 101 (1975) erreicht durch eine schwache Säurebehandlung und/oder Zufügung von Polyglykolen, Zuckern und/oder Polyolen, wobei der Zusatz eines Polyglykols und Zuckers unumgänglich ist.

Die Entfernung der letzten Spuren antikomplementär wirkender Aktivität wird schliesslich durch eine dritte Etappe erreicht, die aus einer selektiven Adsorption an Aluminiumhydroxid besteht.

Die Reihenfolge dieser drei Etappen ist für den Erfolg ausschlaggebend.

Das nach diesem Verfahren erhaltene Immunglobulin weist keine antikomplementäre Aktivität mehr auf und behält seine ursprüngliche Molekularstruktur und auch die unveränderte Antikörperaktivität.

Dieses hochgesteckte Ziel kann nur durch die erfinderische Kombination der drei Verfahrensschritte erreicht werden.

Gegenstand der Erfindung ist demnach ein intravenös verabreichbares humanes Immunglobulin (IgG) zur Verstärkung der Immunabwehr des menschlichen Organismus, dadurch gekennzeichnet, dass es aus nativem, chemisch nichtverändertem und enzymatisch nicht abgebautem, stabilisiertem Immunglobulin besteht, welches keine in vitro nachweisbare antikomplementäre Aktivität enthält und eine unveränderte Antikörperaktivität aufweist.

Das Verfahren zur Herstellung dieses intravenös verabreichbaren Immunglobulins ist dadurch gekennzeichnet, dass man aus humanem Plasma oder Plazenten nach bekannten Methoden gewonnenes, antikomplementäres Immunglobulin an einen Ionenaustauscher bindet, daraus das monomere Immunglobulin mit einer 0.02 bis 0.2 molaren Pufferlösung bei pH 4.0 bis 5.5 selektiv eluiert, konzentriert, durch Behandlung mit einer schwachen Säure vom pH 4.1 bis 4.6 und gegebenenfalls Inkubation bei 30° bis 45°C während ¼ bis zu einer Stunde weitgehend von der antikomplementären Aktivität befreit, danach gegen einen verdünnten Puffer vom pH 5.8 bis 6.1 diafiltriert und/oder durch Zusatz eines Polyethylen- oder Polypropylenglykols und von Saccharose, Lactose, Maltose oder Mannose und/oder von Sorbitol oder Mannitol stabilisiert, wobei der Zusatz eines Polyalkylenglykols und Zuckers unerlässlich ist und anschliessend die letzten Reste von antikomplementärer Aktivität durch Adsorption an Aluminiumhydroxid entfernt.

Im einzelnen besteht das Verfahren darin, dass man

a) nach einer der bekannten Methoden isoliertes Immunglobulin auf einen Ionenaustauscher aufzieht, daraus das monomere Immunglobulin mit einer 0.02 bis 0.2 molaren Pufferlösung, die ein pH von etwa 4.0 bis 5.5 aufweist, eluiert,

b) das weitgehend monomere Eluat nach Konzentrierung diafiltriert oder als schwach saure Lösung vom pH > 4 bis < 5 bei 30 bis 45 Grad Celsius während ca. ¼ bis 1 Stunde inkubiert und diafiltriert, der derart stabilisierten Immunglobulin-Lösung ein Polyglykol, einen Zucker und gegebenenfalls ein Polyol zusetzt und

c) schliesslich ein Aluminiumhydroxid-Gel zufügt, woran die letzten Reste antikomplementärer Aktivität adsorbiert werden, danach das Adsorptionsmittel wieder abtrennt und die erhaltene, stabilisierte Immunglobulin-Fraktion nach bekannten Methoden zu einer lagerfähigen, intravenös injizierbaren Immunglobulin-Darreichungsform verarbeitet.

Als Ausgangsmaterial für das erfinderische Verfahren wird ein Immunglobulin verwendet, welches nach bekannten Fraktionierungsverfahren aus humanem Plasma oder humanen Plazenten gewonnen wurde, beispielsweise nach einem Ethanol-Fraktionierungsverfahren (Cohn et al.), einem Fällungsverfahren mit Salzen (Strauss et al.), Polyethylenglykolen (Polson et al.) oder einem Acridin-Derivat wie Rivanol® (Horeysi et al.) oder einem chromatographischen Verfahren. Als Ausgangsmaterial verwendbares plazentares Immunglobulin wird beispielsweise nach H.L. Taylor et al., J. Amer. chem. Soc. *78*, 1356 (1956) aus dem isotonischen wässrigen Plazentaextrakt durch fraktionierte Fällung mit 95%igem Ethanol erhalten.

Man kann als Ausgangsmaterial Immunglobulin aus normalem oder hyperimmunem humanem Plasma oder aus entsprechenden Plazenten verwenden.

Für die Behandlung von infektiösen Krankheiten und zur Prophylaxe werden Immunglobuline mit spezifischen Antikörpern benötigt.

Man verwendet für solche Indikationen Immunglobuline, welche aus Plasma oder Plazenten gewonnen werden, die Antikörper gegen bestimmte virale oder bakterielle Infektionen enthalten, beispielsweise antivirale Antikörper gegen Hepatitis, Masern, Röteln, Mumps, Tollwut oder antibakterielle Antikörper gegen Tetanus, Diphtherie, Keuchhusten, Staphylokokken, Escherichia Coli, Pseudomonas u.a.m. Für die Behandlung von Morbus haemolytikus neonatorum werden Immunglobuline verwendet aus Plasma oder Plazenten, welche anti-D(Rho) Antikörper enthalten.

Das als Ausgangsmaterial eingesetzte native Immunglobulin wird in einer Proteinkonzentration von 20 bis 40 mg/ml und einem pH von 4.0 bis 5.5 verwendet. Beispielsweise wird eine Cohn-Fraktion II und III, eine Endfällung venöser oder plazentarer Immunglobuline oder ein Lyophilisat durch Auflösung oder eine eventuell sogar Ethanol enthaltende Immunglobulin-Lösung durch entsprechende Verdünnung und durch Säurezusatz auf pH 4.0 bis 5.0 eingestellt. Diese Lösung aus IgG-Monomeren, IgG-Dimeren, IgG-Trimeren und IgG-Polymeren wird mittels eines Ionenaustauschers aufgetrennt (chromatographiert).

Als Ionenaustauscher wird ein Kationenaustauscher bevorzugt. Bewährt haben sich insbesondere Carboxymethylcellulosen (CMC).

Die Ionenaustauscher werden vorerst mit einer 0.01 bis 0.04 molaren Pufferlösung vom pH 4.0 bis 5.0 äquilibriert. Unter diesen Bedingungen wird alles Immunglobulin an den Ionenaustauscher gebunden.

Als Pufferlösung für die Äquilibrierung des Ionenaustauschers und die Eluierung der IgG-Monomeren können beliebige biologische unbedenkliche Puffer wie etwa Acetatpuffer, Phosphatpuffer, Citratpuffer oder Aminosäurepuffer verwendet werden. Bevorzugt werden Acetatpuffer. Die IgG-Monomere werden mit einem 0.02 bis 0.2 molaren Puffer vom pH 4.0 bis 5.5, der zusätzlich 0.05 bis 0.15 mol Kochsalz enthält, eluiert.

Die IgG-Aggregate und zwar sowohl die Polymere als auch die Trimere und Dimere bleiben am Ionenaustauscher gebunden und können später mit hochmolarem Puffer abgelöst werden.

Die antikomplementäre Aktivität der monomeren IgG-Fraktion ist schwach, nämlich 60 bis 80 mg/ml/2CH$_{50}$ im Vergleich zum Ausgangsmaterial (0.3 bis 1.0 mg/ml/2CH$_{50}$). Die IgG-Monomeren-Lösung wird konzentriert und bei einem pH von vorzugsweise 4.1 bis 4.6 bei 30 bis 45 Grad Celsius während ¼ bis zu einer Stunde inkubiert. Die Inkubation kann ausgelassen werden, wenn das Ausgangsmaterial bereits eine relativ geringe antikomplementäre Aktivität aufweist.

Die Immunglobulin-Lösung wird nun gegen eine Pufferlösung vom pH 5.8 bis 61 beispielsweise gegen einen 0.005 bis 0.015 molaren Phosphatpuffer diafiltriert. Citrat-, Phthalat- und Aminosäure-puffer könnten dazu ebenfalls verwendet werden. Der durch Diafiltration gereinigten IgG-Lösung wird zur weiteren Stabilisierung ein Polyglykol, ein Zucker und gegebenenfalls ein Polyol beigefügt. Als Polyglykol verwendet man vorzugsweise 0.05 bis 0.3% (Gew/Vol) eines Polyethylenglykols (PEG 1000 bis 6000) oder ein Polypropylenglykol, als Zucker 3 bis 7% (Gew/Vol) Saccharose, Lactose, Maltose oder Mannose, wobei Saccharose gewöhnlich bevorzugt wird, als Polyol 0 bis 7% (Gew/Vol) Sorbitol oder Mannitol. Die erhaltene Lösung wird auf pH 6.4 bis 6.8 eingestellt.

Der dritte Verfahrensschritt ist zur Entfernung der letzten Spuren antikomplementärer Aktivität unentbehrlich. Dazu werden der neutralisierten IgG-Lösung 5 bis 20% (Gew/Gew) Aluminiumhydroxid-Gel zugesetzt. Die Suspension wird 20 Minuten bis 2 Stunden bei Raumtemperatur oder über Nacht bis 4 Grad Celsius gerührt, danach bei etwa 10 000 × g zentrifugiert und sterilisiert. Das Filtrat wird in Endbehälter, beispielsweise Ampullen oder Serumfläschchen, abgefüllt und anschliessend lyophilisiert.

Das so hergestellte Immunglobulin zeigt nach Auflösen des Lyophilisates in destilliertem Wasser oder 0.05 molarer Kochsalzlösung folgende Eigenschaften.

1. Es enthält keine messbare antikomplementäre Aktivität mehr.

2. Der Anteil an monomerem Immunglobulin (IgG) bestimmt durch Gelchromatographie, liegt zwischen 85 bis 95%. Fragmente sind nicht vorhanden.

3. In der Immunoelektrophorese gegen antihuman Antiserum ist nur eine einzige IgG-Präzipitationslinie sichtbar. Spaltprodukte sind nicht nachweisbar.

4. Die Verteilung der IgG-Subklassen des norlmalen Serums bleibt erhalten.

5. Die Aktivität des Antikörperspektrums bleibt, bezogen auf die Proteinkonzentration, unverändert.

Vergleich der antikomplementären Aktivität ausgedrückt in $(mg/ml/2CH_{50})$ von im Handel befindlichen intravenös applizierbaren Immunglobulin-Präparaten mit dem erfindungsgemäss erhaltenen natürlichen, nicht veränderten und nicht abgebauten, stabilisierten Immunglobulin: Die antikomplementäre Aktivität wird angegeben in mg Protein pro ml, die erforderlich sind, um zwei Einheiten Komplement zu hemmen. Sie wird bestimmt durch die 50%ige Hämolyse von sensibilisierten Schaf-Erythrozyten $(mg/ml/2CH_{50})$ nach dem Standardverfahren von M.M. Mayer, Experimental Immunochemistry, 2nd Ed. Seite 133-240, Charles C. Thomas, Springfield sowie US Department of Health, Education and Welfare Public Health Monograph No. 74, "Standardized diagnostic complement fixation method and adaptation to micro test".

Tabelle siehe Seite 5.

Erfindungsgemäss hergestellte Immunglobulin-Präparate wurden in Dosen von 2.5 bis 10 g. (pro dosi) an 48 Patienten intravenös verabreicht. Die Präparate zeigten eine gute Verträglichkeit indem keine unerwarteten Nebenwirkungen beobachtet werden konnten. An dieser Vorprüfung nahmen ausschliesslich Patienten teil, welche kein Immundefizit aufwiesen. Auf Grund dieser Erfahrungen ist eine klinische Prüfung an Patienten mit einem angeborenen oder erworbenen Immundefizit, bei welchen die lebensnotwendige Immunglobulintherapie unter Verwendung aller bisherigen Präparate mit einem gewissen Risiko verbunden ist, gerechtfertigt.

Tabelle

| IgG-Präparat | Antikomplementäre Aktivität $(mg/ml/2CH_{50})$ |
|---|---|
| 16% Standard IgG (zur intramuskulären Applikation) | 0.5— 1.5 |
| Durch Adsorption gereinigtes IgG | 3 —30 |
| Mit β-Propiolacton behandeltes IgG | 40 —50 |
| Mit Säuren behandeltes IgG | 60 —80 |
| Mit Plasmin gespaltenes bzw. partiell abgebautes IgG | 20 —50 |
| Mit Pepsin gespaltenes bzw. partiell abgebautes IgG | keine messbare Aktivität |
| Erfindungsgemäss hergestelltes IgG (Beispiel 1) Ausgangsmaterial | 0.7 |
| CMC Monomer-Fraktion | 60— 88 |
| IgG-Präparat nach Stabilisierung | 150—200 |
| Endprodukt (Beispiel 1) | keine messbare Aktivität |

Der Fortschritt des erfindungsgemässen Präparates und Verfahrens gegenüber dem Stande der Technik ist offensichtlich.

Beispiel 1

6g lyophilisiertes Immunglobulin (IgG), das nach dem bekannten COHN Verfahren Nr. 9 aus normalem humanem plasma hergestellt wurde, wird in 90 ml destilliertem Wasser aufgelöst, Die Lösung wird nach einer Klarfiltration mit 10% Essigsäure auf pH 4.6 eingestellt und auf eine vorher mit 1 L 0.02 molarem Acetatpuffer pH 4.6 äquilibrierte Carboxymethylcellulosekolonne (CMC-Kolonne) (7 cm lang × 6 cm Durchmesser) geladen. Das IgG wird am Ionenaustauscher adsorbiert und die Kolonne wird mit 500 ml des oben erwähnten Acetatpuffers gewaschen. Das monomere IgG wird dann mit 1.5 L 0.1 molarem Acetatpuffer pH 46 der 8.18 g NaCl im Liter

enthält, eluiert und anschliessend mit einem Ultrafilter auf 50 ml konzentriert. Diese Lösung, die eine Proteinkonzentration von 70 bis 80 mg/ml aufweist, wird bei pH 4.1 während 30 Minuten bei 37 Grad Celsius in einem Wasserbad inkubiert.

Durch Diafiltration gegen 1 L Phosphatpuffer (0.015 M, pH 6.05) wird das pH allmählich auf 5.8 eingestellt. Zur Stabilisierung werden dem Produkt 0.3% (Gew/Vol) PEG 4000 und 7% (Gew/Vol) Saccharose zugefügt. Das pH wird mit NaOH 0.1 N auf 6.4 eingestellt. Nach Zugabe von 10% (Gew/Gew) Aluminiumhydroxidgel wird die Lösung während 30 Minuten bei Raumtemperatur gerührt. Die Suspension wird 20 Minuten bei 10000 × g zentrifugiert und der Übertstand sterilfiltriert. Das Präparat wird in steril in die Endbehälter verteilt und lyophilisiert. Dabei wird in die Endbehälter (Ampullen, Serumfläschchen etc.) soviel IgG-Lösung eingefüllt, dass beim Auflösen des Lyophilisates inbidestilliertem Wasser oder 0.05 M Kochsalzlösung eine 5%ige Protein-(IgG)-Lösung erhalten wird, die sich zur intravenösen Injektion oderzur Infusion eignet.

### Beispiel 2

Man löst, filtriert und reinigt über CMC 6 g lyophilisiertes IgG nach der im Beispiel 1 beschriebenen Methode. Die Monomerenfraktion wird nach Konzentration, ohne Säurebehandlung, direkt gegen den 0.015 molaren Phosphatpuffer vom pH 6.05 diafiltriert, bis das pH der Lösung 5.8 erreicht. Nach Zugabe von 0.5% PEG 4000 und je 3.5% Maltose und Mannitol zur Lösung wird das pH auf 6.4 eingestellt und mit 10% (Gew/Gew) Aluminiumhydroxidgel gemischt. Die Suspension wird während 15 Stunden bei 4 Grad Celsius gerührt, zentrifugiert, filtriert und wie im Beispiel 1 weiterverarbeitet.

### Beispiel 3

Als Ausgangsmaterial wird die Endfällung aus dem COHN-Verfahren Nr. 9, die noch Ethanol enthält, verwendet. Das Präzipitat wird in eiskaltem Wasser gelöst und nach Beispiel 1 weiterverarbeitet, wobei aber die Auftrennung an der CMC-Kolonne bei 4 Grad Celsius durchgeführt wird. Die übrigen Operationen werden wie im Beispiel 1 durchgeführt. Anstelle von Saccharose wird die gleiche Menge Lactose zugesetzt.

Das Endprodukt hat dieselben vorteilhaften Eigenschaften wie das Endprodukt von Beispiel 1.

### Beispiel 4

Als Ausgangsmaterial wird lyophilisiertes IgG, das nach dem COHN-Verfahren Nr. 9 aus humanem hyperimmunem Plasma mit hohem spezifischem anti-Tetanus Titer hergestellt wurde, eingesetzt. Das Ausgangsmaterial enthielt bei 10% Proteinkonzentration 280 I.E. Te/ml. Die wässrige Auflösung dieses Lyophilisates enthaltend 30 mg Protein/ml wird nach dem im Beispiel 1 beschriebenen 3 stufigen Verfahren getrennt, gereinigt und stabilisiert. Das Produkt ist frei von antikomplementärer Aktivität und enthält als 5% Proteinlösung 150 IE Tetanus/ml.

### Beispiel 5

Als Ausgangsmaterial wird spezifische anti-D-Antikörper (150 mcg/ml in einer 10%igen Proteinlösung) enthaltendes IgG eingesetzt, welches nach Beispiel 1 bearbeitet wird. Das Endprodukt enthält als 5%ige IgG-Lösung 85 mcg/ml spezifische anti-D-Antikörper und ist frei von antikomplementärer Aktivität.

### Beispiele 6 bis 12

Analog wie in den Beispielen 4 und 5 beschrieben, werden auch intravenös optimal verträgliche Präparate erhalten, welche die nachfolgend aufgeführten spezifischen Antikörper enthalten.

6. anti-Diphtherie Antikörper.
7. anti-Hepatitis-B Antikörper.
8. anti-Röteln Antikörper.
9. anti-Mumps Antikörper.
10. anti-Tollwut Antikörper.
11. anti-Masern Antikörper.
12. anti-B. Pertussis Antikörper.

Diese Produkte behalten — bezogen auf den Proteingehalt — ihre hohe spezifische Antikörperaktivität.

### Beispiele 13 bis 22

Immunglobulin-fraktionen, die aus isotonischen wässrigen Placetaextrakten durch Fällung mit 95%igem Ethanol gewonnen wurden, werden auf eine Proteinkonzentration von 30 mg/ml und ein pH von 5.0 eingestellt und nach dem im Beispiel beschriebenen Verfahren getrennt, gereinigt und stabilisiert. Die Produkte sind frei von antikomplementärer Aktivität und damit intravenös optimal verträglich und enthalten das im Ausgangsmaterial vorhandene Antikörperspektrum.

13. IgG mit unspezifischem Antikörperspektrum.
14. IgG mit anti-Röteln Antikörper.
15. IgG mit anti-Tetanus Antikörper.
16. IgG mit anti-D Antikörper.
17. IgG mit anti-Diphtherie Antikorper.
18. IgG mit anti-Hepatitis Antikörper.
19. IgG mit anti-Mumps Antikörper.
20. IgG mit anti-Tollwut Antikörper.
21. IgG mit anti-Masern Antikörper.
22. IgG mit anti-B. Pertussis Antikörper.

### Patentansprüche

1. Intrevenös verabreichbares humanes Immunglobulin (IgG) zur Verstärkung der Immunabwehr des menschlichen Organismus, dadurch gekennzeichnet, dass es aus nativen, chemisch nicht verändertem und enzymatisch nicht abgebautem, stabilisiertem Immunglobulin besteht, welches, bestimmt nach dem in Spalte 7, Zeilen 47—64 angegebenen Standardverfahren von M.M. Mayer, keine in vitro nachweisbare antikomplementäre Aktivität enthält und eine unveränderte Antikörperaktivität aufweist.

2. Verfahren zur Herstellung von intravenös verabreichbarem Immunglobulin zur Verstärkung der Immunabwehr des menschlichen Organismus, dadurch gekennzeichnet, dass man aus humanem Plasma oder Plazenten nach bekannten Methoden gewonnenes, antikomplementäres Immunglobulin an einen Ionenaustauscher bindet, daraus das monomere Immunglobulin mit einer 0.02 bis 0.2 molaren Pufferlösung bei pH 4.0 bis 5.5 selektiv eluiert, konzentriert, durch Behandlung mit einer schwachen Säure vom pH 4.1 bis 4.6 und gegebenenfalls Inkubation bei 30° bis 45°C während ¼ bis zu einer Stunde weitgehend von der antikomplementären Aktivität befreit, danach gegen einen verdünnten Puffer vom pH 5.8 bis 6.1 diafiltriert und/oder durch Zusatz eines Polyethylen- oder Polypropylenglykols und von Saccharose, Lactose, Maltose oder Mannose und/oder von Sorbitol oder Mannitol stabilisiert, wobei der Zusatz eines polyalkylenglykols und Zuckers unerlässlich ist und anschliessend die letzten Reste von antikomplementärer Aktivität durch Adsorption an Aluminiumhydroxid entfernt.

3. Verfahren nach Anspruch 2, dadurch gekennzeichnet, dass man Immunglobulin aus normalem oder hyperimmunem humanem Plasma oder aus entsprechenden Plazenten verwendet.

4. Verfahren nach Anspruch 3, dadurch gekennzeichnet, dass man Immunglobulin verwendet, welches Antikörper gegen bestimmte virale oder bakterielle Krankheitserreger oder anti-D-Antikörper enthält.

**Revendications**

1. Immunoglobuline (IgG) humaine pour administration intraveineuse destinée à renforcer les défenses immunitaires de l'organisme humain, caractérisée en ce qu'elle consiste en immunoglobuline native, stabilisée, chimiquement non modifiée et enzymatiquement non dégradée, qui déterminée selon le procédé standard de M.M. Mayer indiqué en colonne 7, lignes 47—64, ne contient pas d'activité anticomplémentaire démontrable in vitro et présente un activité d'anticorps inchangée.

2. Procédé de préparation d'immunoglobuline pour administration intraveineuse destinée à renforcer les défenses immunitaires de l'organisme humain, caractérisé en ce qu'une immunoglobuline anticomplémentaire obtenue par des méthodes connues à partir de plasma humain ou de placentas est fixée sur un échangeur d'ions, l'immunoglobuline monomère en est éluée sélectivement avec une solution tampon 0.02 à 0.2 molaire, pH 4.4 à 5.5, concentrée, débarrassée en grande partie de l'activité anticomplémentaire par traitement avec un acide faible pH 4.1 à 4.6 et éventuellement incubation à 30 à 45°C pendant ¼ d'heure à une heure, puis dialysée contre un tampon dilué pH 5.8 à 6.1 et/ou stabilisée par addition d'un polyéthylène glycol ou un polypropylène glycol et de saccharose, lactose, maltose ou mannose et/ou de sorbitol ou mannitol, l'addition d'un polyalkylène glyco et d'un sucre étant indispensable et finalement les derniers restes d'activité anticomplémentaire sont éliminés par adsorption sur hydroxyde d'aluminium.

3. Procédé selon la revendication 2, caractérisé en ce qu'on utilise une immunoglobuline obtenue à partir de plasma humain normal ou hyperimmun ou à partir de placentas correspondants.

4. Procédé selon la revendication 3, caractérisé en ce qu'on utilise une immunoglobuline qui contient des anticorps dirigés contre certains agents pathogènes d'origine virale ou bactérienne ou des anticorps anti-D.

**Claims**

1. Intravenously administerable human immune globulin (IgG) for strengthening the immune defences of the human organism, characterised in that it consists of native, stabilised immune globulin not modified chemically and not degraded enzymatically, which, determined according to the standard method of M.M. Meyer as specified in column 7, lines 47—64, does not have any anticomplementary activity detectable in vitro and has an unmodified antibody activity.

2. Process for the production of intravenously administerable immune globulin for strenghtening the immune defences of the human organism, characterised in that anticomplementary immune globulin obtained by known methods from human plasma or placentas is combined with an ion exchanger, the monomeric immune globulin is selectively eluted therefrom with an 0.02 to 0.2 molar buffer solution with apH of 4.0 to 5.5, concentrated, substantially freed of the anti-complementary activity by treatment with a weak acid with a pH of 4.1 to 4.6 and possibly incubation at 30° to 45°C for ¼ to one hour, then diafiltered against a diluted buffer with a pH of 5.8 to 6.1, and/or stabilised by addition of a polyethylene glycol or polypropylene glycol and of saccharose, lactose, maltose or mannose and/or of sorbitol or mannitol, the addition of a polyalkylene glycol and sugar being indispensable, and then the last residues of anticomplementary activity are removed by adsorption on aluminium hydroxide.

3. Process according to claim 2, characterised in that immune globulin from normal or hyperimmune human plasma or from corresponding placentas is used.

4 Process according to claim 3, characterised in that immune globulin is used which contains antibodies against specific viral or bacterial pathogenic agents or anti-D antibodies.